# EUROPÄISCHE PATENTSCHRIFT

(11) **EP 1 115 858 B1**
(45) Veröffentlichungstag und Bekanntmachung des Hinweises auf die Patenterteilung: **02.04.2003**
(21) Anmeldenummer: 99948807.5
(22) Anmeldetag: 21.09.1999
(51) Int. Cl.: C12N 15/11, C07H 21/00, A61K 31/7088, C12Q 1/68, A01K 67/027, A61P 31/00

(54) **ANTISENSE-SEQUENZEN FÜR DIE HEMMUNG DER EXPRESSION DES ADHÄSIONSMOLEKÜLS ICAM-1**
ANTISENSE-SEQUENCES FOR INHIBITING THE EXPRESSION OF THE ADHESION MOLECULE ICAM-1
SEQUENCES ANTI-SENS DESTINEES A L'INHIBITION DE L'EXPRESSION DE LA MOLECULE D'ADHESION ICAM-1

(30) Priorität: 25.09.1998 DE 19844111; 04.12.1998 DE 19856138; 08.06.1999 DE 19926110
(43) Veröffentlichungstag der Anmeldung: 18.07.2001
(73) Patentinhaber: Deutsches Krebsforschungszentrum Stiftung des öffentlichen Rechts, 69120 Heidelberg (DE)
(72) Erfinder: PATZEL, Volker, D-63457 Hanau (DE); KRONENWETT, Ralf, D-69120 Heidelberg (DE); STEIDL, Ulrich, D-69121 Heidelberg (DE); HAAS, Rainer, D-69121 Heidelberg (DE); SCZAKIEL, Georg, D-69181 Leimen (DE)
(74) Vertreter: Müller-Boré & Partner Patentanwälte
(86) Internationale Anmeldenummer: EP9906972
(87) Internationale Veröffentlichungsnummer: WO00018907

(56) Entgegenhaltungen:
- WO-A-94/05333
- WO-A-95/28412
- WO-A-98/24797
- LEE CH. ET AL.: "Antisense gene suppression against human ICAM-1, ELAM-1, and VCAM-1 in cultured human umbilical vein endothelial cells." SHOCK 1995 JUL;4(1):1-10, XP000892942
- BENNETT CF. ET AL.: "An ICAM-1 antisense oligonucleotide prevents and reverses dextran sulfate sodium-induced colitis in mice." J PHARMACOL EXP THER 1997 FEB;280(2):988-1000, XP002134404 in der Anmeldung erwähnt
- GEMMELL E. ET AL.: "Adhesion molecule expression in chronic inflammatory periodontal disease tissue." J PERIODONTAL RES 1994 JAN;29(1):46-53, XP000891655
- CHIANG, M.-Y. ET AL.: "Antisense oligonucleotides inhibit intercellular adhesion molecule-1 expression by two distinct mechanisms" JOURNAL OF BIOLOGICAL CHEMISTRY., Bd. 266, 25. September 1991 (1991-09-25), Seiten 18162-18171, XP002134405 ISSN: 0021-9258 in der Anmeldung erwähnt
- PATZEL V. ET AL.: "Theoretical design of antisense RNA structures substantially improves annealing kinetics and efficacy in human cells " NAT BIOTECHNOL 1998 JAN;16(1):64-8, XP002085109
- PATZEL V. ET AL.: "A theoretical approach to select effective antisense oligodeoxyribonucleotides at high statistical probability." NUCLEIC ACIDS RES 1999 NOV 15;27(22):4328-34, XP002134406

### SEQUENZPROTOKOLL

<110> Deutsches Krebsforschungszentrum
<120> Antisense-Sequenzen für die Hemmung der Expression des Adhäsionsmoleküls ICAM-1
<130> ICAM-1 Antisense-Oligonukleotide

<140>
<141>

<150> 19844111.8
<151> 1998-09-25

<150> 19856138.5
<151> 1998-12-04

<150> 19926110.5
<151> 1999-06-08

<160> 37
<170> PatentIn Ver. 2.1

<210> 1
<211> 20
<212> DNA
<213> Homo sapiens

<400> 1
   ggcgtggctt gtgtgttcgg 20

<210> 2
<211> 20
<212> DNA
<213> Homo sapiens

<400> 2
   gcttgtgtgt tcggtttcat 20

<210> 3
<211> 20
<212> DNA
<213> Homo sapiens
<400> 3
   cggtttcatg ggggtccctt 20

<210> 4
<211> 20
<212> DNA
<213> Homo sapiens

<400> 4
   ttcggtttca tgggggtccc 20

<210> 5
<211> 20
<212> DNA
<213> Homo sapiens

<400> 5
   cgtggcttgt gtgttcggtt 20

<210> 6
<211> 20
<212> DNA
<213> Homo sapiens

<400> 6
   gaggcgtggc ttgtgtgttc 20

<210> 7
<211> 20
<212> DNA
<213> Homo sapiens

<400> 7
   ggaggcgtgg cttgtgtgtt 20

<210> 8
<211> 20
<212> DNA
<213> Homo sapiens

<400> 8
   gggaggcgtg gcttgtgtgt 20

<210> 9
<211> 20
<212> DNA
<213> Homo sapiens

<400> 9
   agggaggcgt ggcttgtgtg 20

<210> 10
<211> 20
<212> DNA
<213> Homo sapiens

<400> 10
   cagggaggcg tggcttgtgt 20

<210> 11
<211> 20
<212> DNA
<213> Homo sapiens

<400> 11
   ttcagggagg cgtggcttgt 20

<210> 12
<211> 20
<212> DNA
<213> Homo sapiens

<400> 12
   gttcggtttc atgggggtcc 20

<210> 13
<211> 20
<212> DNA
<213> Homo sapiens

<400> 13
   ctgggagcca tagcgaggct 20

<210> 14
<211> 20
<212> DNA
<213> Homo sapiens

<400> 14
   gggagccata gcgaggctga 20

<210> 15
<211> 20
<212> DNA
<213> Homo sapiens

<400> 15
   gagccatagc gaggctgagg 20

<210> 16
<211> 20
<212> DNA
<213> Homo sapiens

<400> 16
   gcacgagaaa ttggctccat 20

<210> 17
<211> 20
<212> DNA
<213> Homo sapiens

<400> 17
   aaattggctc catggtgatc 20

<210> 18
<211> 20
<212> DNA
<213> Homo sapiens

<400> 18
   catggtgatc tctcctcacc 20

<210> 19
<211> 20
<212> DNA
<213> Homo sapiens

<400> 19
   ggctccatgg tgatctctcc 20

<210> 20
<211> 20
<212> DNA
<213> Homo sapiens

<400> 20
   cgagaaattg gctccatggt 20

<210> 21
<211> 20
<212> DNA
<213> Homo sapiens

<400> 21
   cacgagaaat tggctccatg 20

<210> 22
<211> 20
<212> DNA
<213> Homo sapiens

<400> 22
   ggcacgagaa attggctcca 20

<210> 23
<211> 20
<212> DNA
<213> Homo sapiens

<400> 23
   gtgcggcacg agaaattggc 20

<210> 24
<211> 20
<212> DNA
<213> Homo sapiens

<400> 24
   ctcccgggtc tggttcttgt 20

<210> 25
<211> 20
<212> DNA
<213> Homo sapiens

<400> 25
   gaagctcccg ggtctggttc 20

<210> 26
<211> 20
<212> DNA
<213> Homo sapiens

<400> 26
   agtcactgat tccccgatgg 20

<210> 27
<211> 20
<212> DNA
<213> Homo sapiens

<400> 27
   tgattccccg atgggcagtg 20

<210> 28
<211> 20
<212> DNA
<213> Homo sapiens

<400> 28
   cagtcactga ttccccgatg 20

<210> 29
<211> 20
<212> DNA
<213> Homo sapiens

<400> 29
   gatgcgtggc ctagtgtttt 20

<210> 30
<211> 20
<212> DNA
<213> Homo sapiens

<400> 30
   atcagatgcg tggcctagtg 20

<210> 31
<211> 45
<212> DNA
<213> Homo sapiens

<400> 31
   aagggacccc catgaaaccg aacacacaag ccacgcctcc ctgaa 45

<210> 32
<211> 24
<212> DNA
<213> Homo sapiens

<400> 32
   cctcagcctc gctatggctc ccag 24

<210> 33
<211> 42
<212> DNA
<213> Homo sapiens

<400> 33
   ggtgaggaga gatcaccatg gagccaattt ctcgtgccgc ac 42

<210> 34
<211> 24
<212> DNA
<213> Homo sapiens

<400> 34
   acaagaacca gacccgggag cttc 24

<210> 35
<211> 27
<212> DNA
<213> Homo sapiens

<400> 35
   cactgcccat cggggaatca gtgactg 27

<210> 36
<211> 24
<212> DNA
<213> Homo sapiens

<400> 36
   aaaacactag gccacgcatc tgat 24

<210> 37
<211> 20
<212> DNA
<213> Homo sapiens

<400> 37
   ggaaagtgcc atcctttaga 20

## Beschreibung

Die vorliegende Erfindung betrifft spezifische Antisense-Nukleinsäuren, welche die Expression des Adhäsionsmoleküls ICAM-1 in Säugerzellen, insbesondere menschlichen Zellen, hemmen, und diese Antisense Nukleinsäuren enthaltende Vektoren und Wirtszellen sowie diese Antisense-Nukleinsäure enthaltende pharmazeutische Zusammensetzungen zur Behandlung von insbesondere akut oder chronisch entzündlichen Erkrankungen, Virusinfektionen, Metastasierung, Entzündungen der Haut, Mobilisierung von hämatopoetischen Stammzellen, Erkältung und alle biologischen Prozesse unter Beteiligung von ICAM-1 auf molekularer Ebene.

Entzündungen sind Reaktionen des vaskularisierten Gewebes auf Reizungen wie zum Beispiel Infektionen und mechanische Verletzungen, Beispiele für entzündliche Erkrankungen sind unter anderem Morbus Crohn, rheumatoide Arthritis und Organabstossungsreaktionen, Virusinfektionen, Metastasierung, Entzündungen der Haut, Mobilisierung von hämatopoetischen Stammzellen, Erkältung und alle biologischen Prozesse unter Beteiligung von ICAM-1 auf molekularer Ebene. Die Infiltration von Leukozyten ist ein wichtiger Schritt bei pathologischen Entzündungsreaktionen, wobei Zell-Zell-Adhäsion hierfür den initialen Schritt darstellt. Adhäsion wird durch Oberflächenproteine auf Leukozyten und der endothelialen Oberfläche in Rezeptor-Ligand-analoger Weise vermittelt. Das endotheiale Oberflächenprotein ICAM-1 nimmt bei solchen Zell-Zell-Kontakten die Rolle des Rezeptors für die Leukozytenliganden CD11a/CD18, auch LFA1 genannt, und CD11b/CD18, auch Mac-1 genannt, ein. Monoklonale Antikörper gegen Leukozyten-Adhäsionsmoleküle können die Adhäsion von Leukozyten und Endothelzeilen sowohl *in vitro* als auch *in vivo* hemmen (Arfors et al. 1987 Blood 69:338-340; Vedder et al. 1988 J. Cli. Invest. 81:939-944). Bei verschiedenen Formen akuter und chronischer Entzündungen scheint daher die Hemmung des Adhäsionsmoleküls ICAM-1 erfolgversprechend.

Kausale Therapieansätze bei pathologischen Entzündungsreaktionen mit Hilfe von Substanzen, die auf molekularer Ebene eingreifen, befinden sich gegenwärtig im experimentellen Stadium. Zum Beispiel werden mit gewissem Erfolg sowohl monoklonale Antikörper gegen LFA1 getestet als auch ICAM-1-gerichtete Antisense-Oligonukleotide (Yacyshyn et al., 1998 Gastroenterology 114: 1133-1142; Stepkowski et al. 1995 Transplant. Proc. 27: 113; Kavanaugh et al., 1994 Arthritis-Reum. 37: 992-999; Arfors et al. 1987 Blood 69:338-340; Vedder et al. 1988 J. Cli. Invest. 81-939-944; Bennett et al. 1997 J. Pharmacol. Exp. Ther. 280:988-1000). Nukleinsäuren als therapeutische Moleküle haben grundsätzliche Vorteile gegenüber anderen Substanzklassen wie zum Beispiel Peptiden und Proteinen. Hierzu zählen ihre geringe oder nicht vorhandene Immunogenität bzw. Nebenwirkungen und Toxizität.

Da die Auswahl der Antisense-Sequenzen bislang eher willkürlich und ohne rationale Auswahlkriterien erfolgte, war ein großer Teil der untersuchten Sequenzen nicht effektiv. Die Effektivität von Antisense-Inhibitoren in lebenden Systemen wird durch das Assoziationsverhalten *in vitro* reflektiert. Wichtige Einflußgrößen auf das Assoziationsverhalten zwischen der Antisense-Nukleinsäure und der Target-RNA, wie beispielsweise die Zugänglichkeit der Target-RNAs für die Antisense-Nukleinsäuren, wurden nicht berücksichtigt. Ferner wurden bei den Antisense-RNAs die strukturelle Vielfalt nicht berücksichtigt und damit das Potential von effektiven Antisense-Inhibitoren nicht ausgeschöpft.

Somit liegt der vorliegenden Erfindung die Aufgabe zugrunde, Antisense-Nukleinsäuren bereitzustellen, die eine besonders wirkungsvolle Hemmung der ICAM-1-Genexpression in Säugerzellen aufweisen.

Diese Aufgabe wird durch die in den beigefügten Patentansprüchen gekennzeichneten Ausführungsformen gelöst. Insbesondere wird eine Antisense-Nukleinsäure bereitgestellt, welche gegen eine spezifische RNA-Sequenz von ICAM-1 als Target bzw. Zielmolekül gerichtet ist und mindestens eine Sequenz, ausge-wählt aus SEQ ID NO 1-12, 15, 29 und 30, enthält.

Der Begriff "Antisense-Nukleinsäure" bedeutet ein natives, halbsynthetisches, synthetisches oder modifiziertes Nukleinsäuremolekül aus Desoxyribonukleotiden und/oder Ribonukleotiden und/oder modifizierten Nukleotiden.

Die lokale Zielsequenzen im ICAM-1-Gen umfassen die Positionen 1627-1671 (SEQ ID NO 31; Antisense-Nukleinsäuren mit den SEQ ID NO 1-12), 59-82 (SEQ ID NO 32; Antisense-Nukleinsäure mit der SEQ ID NO 15) oder 1851-1874 (SEQ ID NO 36; Antisense-Nukleinsäuren mit den SEQ ID NO 29 und 30).

Die erfindungsgemäßen Antisense-Nukleinsäuresequenzen zeigen eine signifikant stärkere Hemmung als die vier wirksamsten bekannten Antisense-Oligonukleotide der Fa. ISIS Inc., ("ISIS1570", "ISIS2302", "ISIS1939" und "ISIS3067") und sind daher ausgezeichnete Inhibitoren der ICAM-1 Expression in lebenden Zellen.

Ein weiterer Gegenstand der vorliegenden Erfindung ist ein Vektor, der die vorstehend definierte, erfindungsgemäße Antisense-Nukleinsäure oder der eine entsprechende, zur Antisense-Nukleinsäure komplementäre DNA-Sequenz enthält, die nach einer Transkription in geeigneten Wirtszellen zur vorstehend definierten, erfindungsgemäßen Antisense-Nukleinsäure führt. Der erfindungsgemäße Vektor kann vorzugsweise geeignete regulatorische Elemente, wie Promotoren, Enhancer, Terminationssequenzen, oder virale Sequenzen enthalten. In einer erfindungsgemäßen Ausführungsform kann der Vektor beispielsweise (i) zur stabilen Integration der erfindungsgemäßen Nukleinsäure in das genetische Material einer Wirtszelle verwendet werden und/oder (ii) für erhöhte zelluläre Aufnahme geeigent sein und/oder (iii) nukleäre Importsignale enthalten und/oder (iv) zum Zell-Zell-Transport geeignet sein.

Ein weiterer Gegenstand der vorliegenden Erfindung ist eine Wirtszelle, welche die erfindungsgemäße Antisense-Nukleinsäure oder den erfindungsgemäßen Vektor enthält. Geeignete Wirtszellen sind beispielsweise Säugerzellen, insbesondere menschliche Zellen.

Ein weiterer Gegenstand der vorliegenden Erfindung ist eine pharmazeutische Zusammensetzung, welche die erfindungsgemäße Antisense-Nukleinsäure oder ein Gemisch von mindestens zwei erfindungsgemäßen Antisense-Nukleinsäuren oder den erfindungsgemäßen Vektor, gegebenfalls in einem im Stand der Technik bekannten, pharmazeutisch verträglichen Träger und/oder Verdünnungsmittel enthält. Die erfindungsgemäße Zusammensetzung kann zur Hemmung oder Beseitigung von insbesondere akut oder chronisch entzündlichen Krankheitszuständen, beispielsweise Morbus Crohn, rheumatoide Arthritis, Organabstoßungsreaktionen, Virusinfektionen, Metastasierung, Entzündungen der Haut, Mobilisierung von hämatopoetischen Stammzellen, Erkältung und alle biologischen Prozesse unter Beteiligung von ICAM-1 auf molekularer Ebene, Colitis ulcerosa, Rheumatoide Arthritis, Lupus erythematodes, "Graft-versus-host Reaktionen" nach allogener Knochenmarkstransplantation, Psoriasis, Asthma, Neurodermitis durch transiente oder stabile Integration der erfindungsgemäßen Antisense-Nukleinsäure mittels Transformation bzw. Transfektion und anderen im Stand der Technik bekannten Einschleusungsverfahren in ICAM-1 exprimierenden Wirtszellen verwendet werden. Ferner kann die erfindungsgemäße Zusammensetzung auch für kosmetische Zwecke, beispielsweise zur Behandlung von Akne, verwendet werden.

In einer bevorzugten Ausführungsform kann die erfindungsgemäße Zusammensetzung zur Behandlung von Parodontose bzw. entzündlichen Erkrankungen der Mundhöhle, insbesondere bei entzündlichen Zahnfleischerkrankungen verwendet werden. Die Parodontose ist eine weitverbreitete Krankheit der Mundhöhle. Zirka 20% der Bevölkerung leiden an temporären oder chronischen Zahnfleischentzündungen, die auf einer Entzündung des sog. Kontaktepithels des Zahnfleisches beruhen. Bei extremer Ausbildung dieser Entzündungen kann es durch die Einwanderung von Bakterien in das darunter gelegene Mesenchym zur Durchtränkung des Zahns mit Endotoxinen und letztendlich zum Zahnverlust führen. Ursachen für eine starke Entzündung des Kontaktepithels kann eine unkontrollierte Ausbreitung von mikrobiellen Plaque der Bakterienflora in der Mundhöhle sein oder ein Versagen des Immunsystemes zur Hemmung von Entzündungsprozessen. In ersterem Falle wird der Patient durch mechanische Beseitigung des bakteriellen Befalls durch Säuberung des Zahnes und/oder Verabreichung von Antibiotika behandelt. Bei zirka 20% aller Parodontose-Patienten liegt jedoch eine therapieresistente Parodontitis vor, bei der keine parodontalpathogenen Keime nachzuweisen sind. Hier ist die Parodontitis auf eine inadäquate Reaktion des immunsystems auf die natürliche Ausbreitung von Plaquebakterien zurückzuführen.

Bei krankhaften Zahnfleischentzündungen werden bisher vor allem Antibiotika gegen Entzündungserreger mit wechselndem Erfolg lokal verwendet. Eine alternative Therapie bei fortgeschrittenen Stadien der Parodontose stellen sogenannte GTR-Ansätze (Guided Tissue Regeneration) dar, bei denen der Versuch unternommen wird, den durch die Erkrankung verloren gegangene Zahnhalteapparat zu regenerieren. Der Therapieerfolg hängt bei diesem Ansatz entscheidend von der Entzündungsfreiheit während der Reparationsphase ab.

Ferner können die erfindungsgemäßen Antisense-Nukleinsäuren zur Regulation oder Suppression der ICAM-1-Genexpression in Säugerzellen verwendet werden.

Rekombinante Adenoviren können ruhende Zellen mit hoher Effizienz infizieren und werden als virales Vektorsystem zur Einschleusung therapeutischer Gene in menschliche Zellen in der somatischen Gentherapie überprüft und experimentell angewendet. Therapeutische Strategien zielen beispielsweise auf genetische Erkrankungen der Lunge oder benutzen die schnelle Akkumulation adenoviraler Partikel in Lebergewebe. Eine der Haupthürden für die Anwendung dieses Vektorsystems ist mit der schnellen Induktion einer Immunantwort gegen den Vektor verknüpft, der auch die Funktionen des Genes für das Adhäsionsmolekül ICAM-1 einschließt. Zum Beispiel wird Lungengewebe kurz nach der Behandlung mit Adenovektoren von Zellen des Immunsystems infiltriert was zu massiven Entzündungsreaktionen führt. Zur Überwindung der unerwünschten Immunantwort gegen adenovirale Vektoren kann daher die Anwendung von ICAM-1-gerichteten Antisense-Nukleinsäuren während der Behanldung mit adenoviralen Vektoren wesentlich beitragen.

Somit ist ein weiterer Gegenstand der vorliegenden Erfindung die Verwendung der erfindungsgemäßen Antisense-Nukleinsäuren zur Unterdrückung der Immunreaktion gegen gentherapeutisch eingesetzte virale, beispielsweise adenovirale, oder nicht-virale Vektoren in Säugern, insbesondere Mensch.

Ein weiterer Gegenstand der vorliegenden Erfindung ist ein mindestens eine oder mehrere erfindungsgemäße Antisense-Nukleinsäuren enthaltender Kit zur Diagnose von ICAM-1-assoziierten Störungen bzw. Krankheiten von Säugern, insbesondere Mensch, wobei die Antisense-Nukleinsäuren mindestens eine nachweisbare Markierung, beispielsweise an Nukleotide gekoppelte Fluoreszenzfarbstoffe, Biotin oder Digoxygenin oder radioaktive Isotope, enthalten.

Ein weiterer Gegenstand der vorliegenden Erfindung ist ein transgener Organismus bzw. Säuger, welcher mindestens eine in das genetische Material stabil integrierte Nukleinsäure enthält, wobei diese Nukleinsäure nach der Transkription eine RNS-Sequenz, die mindestens eine Sequenz, ausgewählt aus SEQ ID NO 1-12, 15, 29 und 30 und dargestellt als RNS, umfaßt.

Die Figuren zeigen:

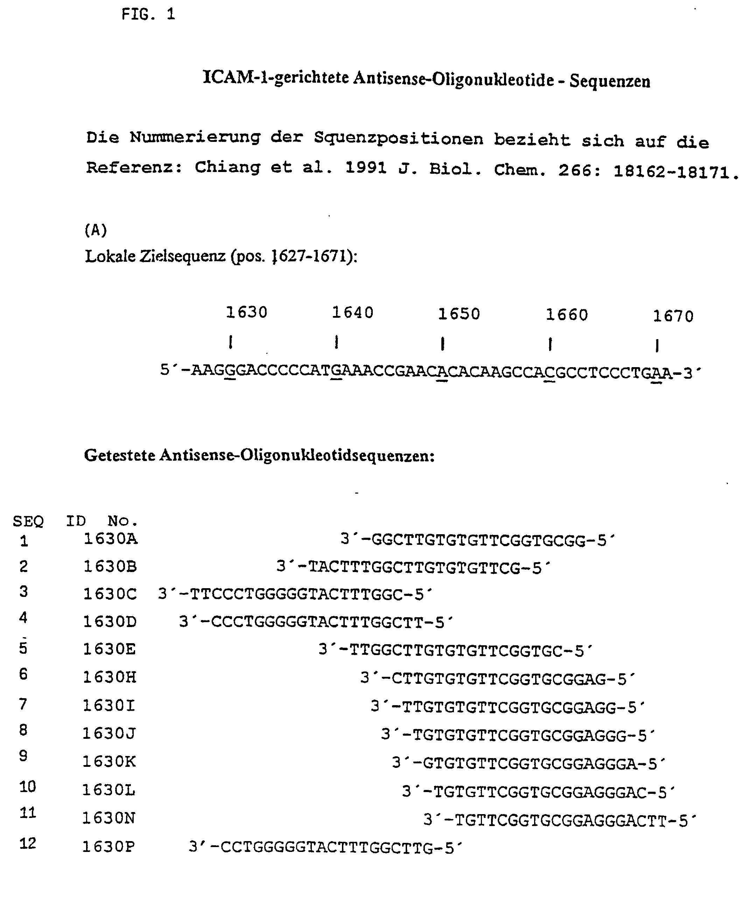

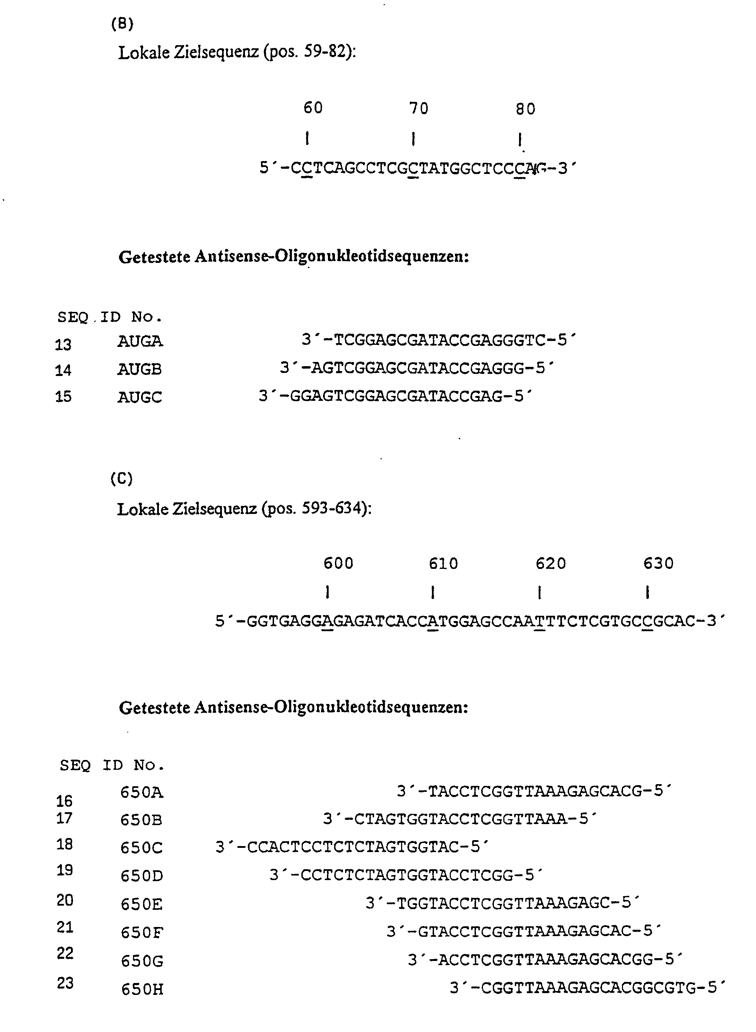

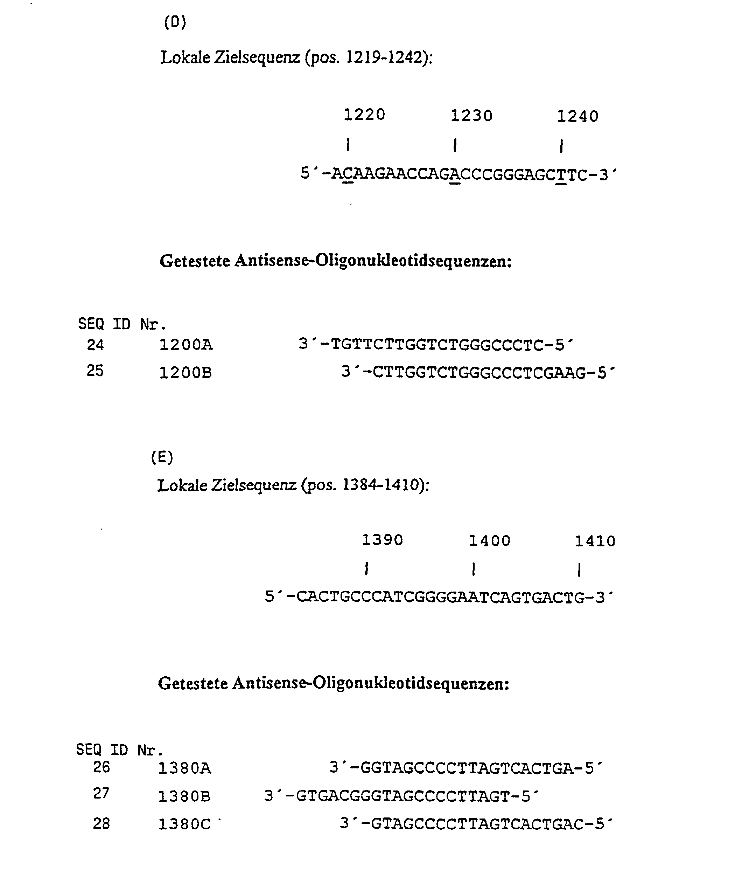

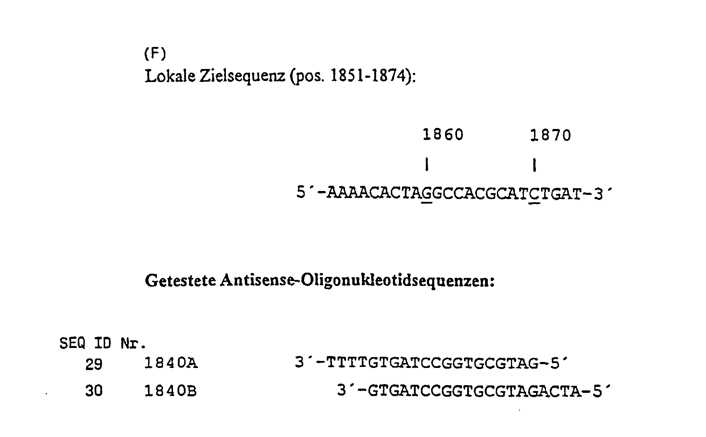

Figur 1 zeigt die erfindungsgemäßen und weitere CAM-1-gerichteten Antisense-Oligonukleotidsequenzen, wobei sich die Nummerierung der Sequenzpositionen auf die Referenz "Chian et al. 1991 J. Biol. Chem. 266: 18162-18171" bezieht. (A) zeigt die Antisense-Oligonukleotide SEQ ID NO 1-12 gegen die lokale ICAM-1-Zielsequenz pos. 1627-1671 (SEQ ID NO 31), (B) zeigt die Antisense-Oligonukleotide SEQ ID NO 13-15 gegen die lokale ICAM-1-Zielsequenz pos. 59-82 (SEQ ID NO 32), (C) zeigt die Antisense-Oligonukleotide SEQ ID NO 16-23 gegen die lokale ICAM-1-Zielsequenz pos. 593-634 (SEQ ID NO 33), (D) zeigt die Antisense-Oligonukleotide SEQ ID NO 24 und 25 gegen die lokale ICAM-1-Zielsequenz pos. 1219-1242 (SEQ ID NO 34), (E) zeigt die Antisense-Oligonukleotide SEQ ID NO 26-28 gegen die lokale ICAM-1-Zielsequenz pos. 1384-1410 (SEQ ID NO 35) und (F) zeigt die Antisense-Oligonukleotide SEQ ID NO 29 und 30 gegen die lokale ICAM-1-Zielsequenz pos. 1851-1874 (SEQ ID NO 36).

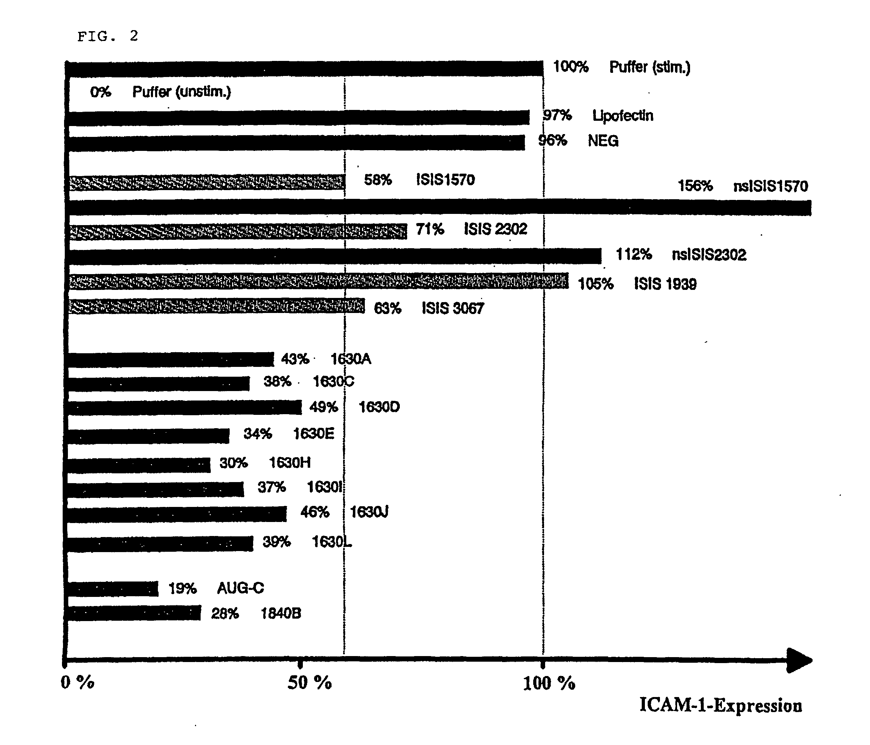

Figur 2 ist eine graphische Darstellung der Hemmung der Genexpression von ICAM-1 in ECV304-Zellen nach Lipofectin-vermittelter Transfektion mit bekannten und erfindungsgemäßen Phosphorothioat-modifizierten Antisense-Oligonukleotiden und Cytokin-Stimulation der ICAM-1 Expression. Es ist deutlich ersichtlich, daß die getesteten erfindungsgemäßen Antisense-Oligonukleotide 1630A, C, D, E, H, I, J und L (SEQ ID NO 1, 3-8 und 10), AUGC (SEQ ID NO 5) und 1840B (SEQ ID NO 30) eine signifikant stärkere Hemmung aufweisen als beispielsweise die bekannten Antisense-Oligonukleotide ISIS1570, ISIS2302, ISIS1939 und ISIS3067 der Fa. ISIS Inc. Als Negativkontrolle "NEG" wurde ein als ungünstig vorhergesagtes ICAM-1-gerichtetes Antisense-Olionukleotid mit der Sequenz 5'-GGAAAGTGCCATCCTTTAGA-3' (SEQ ID NO 37) verwendet. Die Oligonukleotide nslSIS1570 und nsISIS2302 sind von ISIS Inc. verwendete Kontrollen.

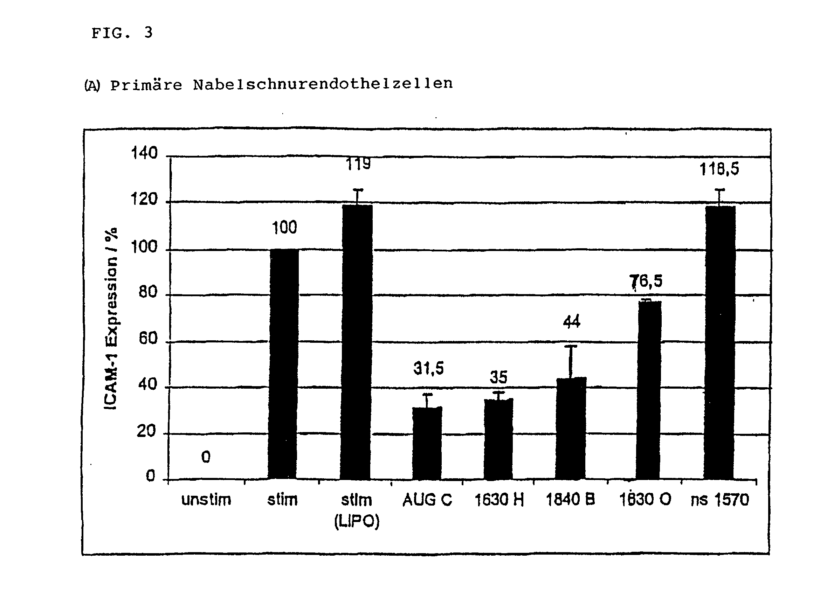

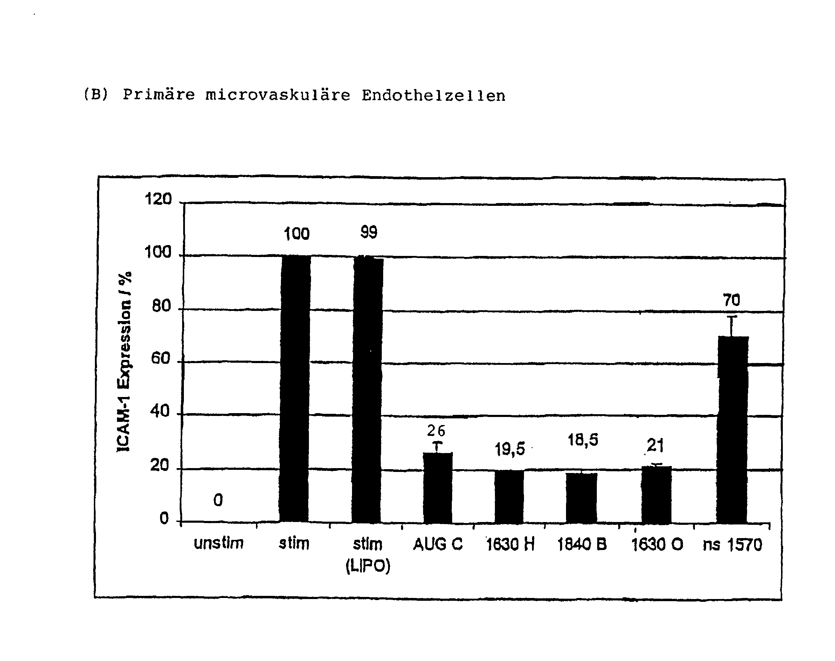

Figur 3 ist eine graphische Darstellung der ICAM-1 Expression in (A) primären Nabelschnurendothelzellen und (B) primären microvaskulären Endothelzellen. "LIPO" bedeutet "Lipofectin"; "unstim" nicht-stimuliert; "stim" stimuliert.

Durch die nachfolgenden Beispiele wird die vorliegende Erfindung näher erläutert.

### Beispiel 1

Ein 60%-80% konfluenter Monolayer der Endothetzellinie ECV304 wurde unter Verwendung des kationischen Lipids DOTMA (LIPOFECTIN, Life Technologies, Karlsruhe) entsprechend der Angaben des Hersteller mit den Oligonukleotiden transfiziert. Dabei wurden Konzentrationen von 0,1 µM Oligonukleotid sowie 5 µg/ml LIPOFECTIN und das Serum-freie Medium OptiMEM (Life Technologies) verwendet. Nach Zugabe des Transfektionsansatzes wurden die Zellen 4 h bei 37°C inkubiert und anschließend das Serum-freie Transfektionsmedium gegen Medium 199 mit 10% FCS ausgetauscht. Nach einer weiteren Inkubationszeit von 4 h wurde das Medium erneut durch Medium 199 mit 10% FCS und 200 U/ml Interleukin 1β ersetzt. Nach einer Stimulationszeit von 16 h bei 37°C wurden die Endothelzellen durch eine 5-minütige Trypsinierung von der Zellkulturschale abgelöst und mit einem Phycoerythrin-gekoppelten, ICAM-1-spezifischen monoklonalen Antikörper gefärbt. Die ICAM-1-Expression wurde als mittlere Fluoreszenzintensität durchflußzytometrisch bestimmt. Das Ausmaß der Hemmwirkung eines Antisense-Oligonukleotids wurde nach folgender Formel berechnet:
[(ICAM-1-Expression-Antisense-Oligonukleotid-behandelter, stimulierter ECV304) - (ICAM-1-Expression unbehandelter, nicht stimulierter ECV304)]/[(ICAM-1-Expression unbehandelter, stimulierter ECV304) - (ICAM-1-Expression unbehandelter, nicht stimulierter ECV304)]

### Beispiel 2

Primäre Nabelschnurendothelzellen bzw. primäre microvaskuläre Endothelzellen (je 5 x 10⁴ Zellen) wurden in 24-Well-Platten kultiviert (Medium: endothelial cell growth medium MV, PromoCell, Heidelberg) und mit Oligonukleotiden transfiziert (100 pmol, Endkonzentration 0,1µM; 10µl Lipofectin [Life Technologies]; 1 ml OPTIMEM-Medium). Nach fünfstündiger Inkubation wurden die Zellen gewaschen, in "endothelial cell growth medium MV" (PromoCell, Heidelberg) für 3 Stunden weiter inkubiert, und die ICAM-1-Expression mit rHu-IL-1β für 18 Stunden stimuliert. Anschließend wurden die Zellen Trypsin-behandelt, mit isotonischem Puffer gewaschen und mit einem PE-konjugierten ICAM-1-gerichteten Antikörper gefärbt. Die Expression von ICAM-1 wurde durch FACS-Analyse quantifiziert. Die Ergebnisse sind für die getesteten Antisense-ODN in den beiden Balkendiagrammen dargestellt (vgl. Figuren 3A und 3B).

## Patentansprüche

1. Antisense-Nukleinsäure, welche gegen eine spezifische Nukleinsäure-Sequenz von ICAM-1 gerichtet ist und aus mindestens einer der in SEQ ID NO 1-12, 15, 29 und 30 **gekennzeichneten** Nukleinsäure-Sequenzen besteht.

2. Antisense-Nukleinsäure nach Anspruch 1, welche Desoxyribonukleotide und/oder Ribonukleotide und/oder modifizierte Desoxyribo- oder Ribonukleotide enthält.

3. Vektor, enthaltend mindestens eine der nach Anspruch 1 oder 2 definierten Antisense-Nukleinsäuren.

4. Wirtszelle, enthaltend mindestens eine nach Anspruch 1 oder 2 definierten Antisense-Nukleinsäuren oder den nach Anspruch 3 definierten Vektor.

5. Pharmazeutische Zusammensetzung, enthaltend die Antisense-Nukleinsäure nach Anspruch 1 oder 2 oder ein Gemisch von mindestens zwei Antisense-Nukleinsäuren nach Anspruch 1 oder 2 oder den Vektor nach Anspruch 3.

6. Verwendung der Antisense-Nukleinsäure nach Anspruch 1 oder 2 oder des Vektors nach Anspruch 3, zur Herstellung eines Medikaments zur Hemmung oder Beseltigung von akut oder chronisch entzündlichen Krankheitszuständen beim Menschen, Virusinfektionen, Metastasierung, Entzündungen der Haut, Mobilisierung von hämatopoetischen Stammzellen, Erkältung und alle biologischen Prozesse unter Beteiligung von ICAM-1 auf molekularer Ebene, Colitis ulcerosa, Rheumatoide Arthritis, Lupus erythematodes, Organabstossungsreaktionen, "Graft-versus-host-Reaktionen" nach allogener Knochenmarkstransplantation, Psoriasis, Asthma, Neurodermitis.

7. Verwendung nach Anspruch 6, wobei die akut oder chronisch entzündlichen Krankheitszustände entzündliche Erkrankungen der Mundhöhle, insbesondere entzündliche Zahnfleischerkrankungen sind.

8. Verwendung der Antisense-Nukleinsäure nach Anpsruch 1 oder 2 oder des Vektors nach Anspruch 3 oder der pharmazeutischen Zusammensetzung nach Anspruch 5 zur Herstellung eines Medikaments zur Regulation oder Suppression der ICAM-1-Genexpression.

9. Verwendung der Antisense-Nukleinsäure nach Anspruch 1 oder 2 oder des Vektors nach Anspruch 3 oder der pharmazeutischen Zusammensetzung nach Anspruch 5 zur Herstellung eines Medikaments zur Unterdrückung der Immunreaktion gegen gentherapeutisch eingesetzte virale oder nicht-virale Vektoren in Säugern.

10. Kit zur Diagnose von ICAM-1-assoziierten Störungen bzw. Krankheiten von Säugern, enthaltend mindestens eine oder mehrere Antisense-Nukleinsäuren nach Anspruch 1 oder 2 mit mindestens einer nachweisbaren. Markierung.

11. Transgener Organismus, welcher mindestens eine in das genetische Material stabil integrierte Nukleinsäure enthält, wobei diese Nukleinsäure nach der Transkription eine RNS-Sequenz, die mindestens eine Sequenz, ausgewählt aus SEQ ID NO 1-12, 15, 29 und 30 und dargestellt als RNS, umfaßt, mit der Maßgabe, daß der transgene Organismus kein Mensch ist.

## Revendications

1. Acide nucléique antisens, qui est dirigé contre une séquence spécifique d'acides nucléiques de ICAM-1 et qui est constitué d'au moins une des séquences d'acides nucléiques **caractérisées** dans SEQ ID N° 1-12, 15, 29 et 30.

2. Acide nucléique antisens suivant la revendication 1, qui contient des désoxyribonucléotides et/ou des ribonucléotides et/ou des désoxyribonucléotides et ribonucléotides modifiés.

3. Vecteur, contenant au moins un des acides nucléiques antisens définis selon la revendication 1 ou 2.

4. Cellule-hôte, contenant au moins un des acides nucléiques antisens définis selon la revendication 1 ou 2 ou le vecteur défini selon la revendication 3.

5. Composition pharmaceutique, contenant l'acide nucléique antisens selon la revendication 1 ou 2 ou un mélange d'au moins deux acides nucléiques antisens selon la revendication 1 ou 2 ou le vecteur selon la revendication 3.

6. Utilisation de l'acide nucléique antisens suivant la revendication 1 ou 2 ou du vecteur suivant la revendication 3 pour la préparation d'un médicament destiné à inhiber ou à prévenir des états pathologiques inflammatoires aigus ou chroniques chez l'homme, des infections virales, la formation de métastases, des inflammations de la peau, la mobilisation de cellules souches hématopoiétiques, le rhume et tous processus biologiques avec la participation de la molécule ICAM-1 au plan moléculaire, la rectocolite hémorragique, l'arthrite rhumatoïde, le lupus érythémateux, des réactions de rejet d'organes, des réactions « graft-versus-host » après transplantation de moelle osseuse allogénique, le psoriasis, l'asthme, la névrodermite.

7. Utilisation suivant la revendication 6, dans laquelle les états pathologiques inflammatoires aigus ou chroniques sont des affections inflammatoires de la cavité buccale, en particulier des inflammations des gencives.

8. Utilisation de l'acide nucléique antisens suivant la revendication 1 ou 2 ou du vecteur suivant la revendication 3 ou de la composition pharmaceutique suivant la revendication 5 pour la préparation d'un médicament destiné à la régulation ou à la suppression de l'expression du gène de la molécule ICAM-1.

9. Utilisation de l'acide nucléique antisens suivant la revendication 1 ou 2 ou du vecteur suivant la revendication 3 ou de la composition pharmaceutique suivant la revendication 5 pour la préparation d'un médicament destiné à la suppression de la réaction immunitaire contre des vecteurs viraux ou non viraux utilisés en thérapie génique chez des mammifères.

10. Kit destiné au diagnostic de troubles ou de maladies associés à la molécule ICAM-1 chez des mammifères, contenant au moins un ou plusieurs acides nucléiques antisens suivant la revendication 1 ou 2 avec au moins un marquage détectable.

11. Organisme transgénique, qui contient au moins un acide nucléique intégré de façon stable au matériel génétique, cet acide nucléique comprenant après la transcription une séquence d'acide ribonucléique qui comprend au moins une séquence choisie entre SEQ ID N° 1-12, 15, 29 et 30 et représentée comme ARN, sous réserve que l'organisme transgénique ne soit pas un être humain.

## Claims

1. Antisense nucleic acid which is directed against a specific nucleic acid sequence of ICAM-1 and consists of at least one of the nucleic acid sequences **characterized in** SEQ ID NO 1-12, 15, 29 and 30.

2. Antisense nucleic acid according to Claim 1, which contains deoxyribonucleotides and/or ribonucleotides and/or modified deoxyribo- or ribonucleotides.

3. Vector containing at least one of the antisense nucleic acids defined according to Claim 1 or 2.

4. Host cell containing at least one of the antisense nucleic acids defined according to Claim 1 or 2, or the vector defined according to Claim 3.

5. Pharmaceutical composition containing the antisense nucleic acid according to Claim 1 or 2 or a mixture of at least two antisense nucleic acids according to Claims 1 or 2 or the vector according to Claim 3.

6. Use of the antisense nucleic acid according to Claim 1 or 2 or of the vector according to Claim 3 for producing a medicament for inhibiting or eliminating acute or chronic inflammatory pathological states in humans, viral infections, metastasis, inflammations of the skin, mobilization of haematopoietic stem cells, coryza and all biological processes involving ICAM-1 at the molecular level, ulcerative colitis, rheumatoid arthritis, lupus erythematosus, organ rejection reactions, graft-versus-host reactions for allogeneic bone marrow transplantation, psoriasis, asthma, neurodermatitis.

7. Use according to Claim 6, where the acute or chronic inflammatory pathological states are inflammatory disorders of the oral cavity, in particular inflammatory gingival disorders.

8. Use of the antisense nucleic acid according to Claim 1 or 2 or of the vector according to Claim 3 or of the pharmaceutical composition according to Claim 5 for producing a medicament for the regulation or suppression of ICAM-1 gene expression.

9. Use of the antisense nucleic acid according to Claim 1 or 2 or of the vector according to Claim 3 or of the pharmaceutical composition according to Claim 5 for producing a medicament for diminishing the immune response to viral or nonviral vectors employed for gene therapy in mammals.

10. Kit for diagnosing ICAM-1-associated disorders or diseases in mammals, containing at least one or more antisense nucleic acid according to Claim 1 or 2 with at least one detectable label.

11. Transgenic organism which contains at least one nucleic acid stably integrated into the genetic material, where this nucleic acid comprises after transcription an RNA sequence which [lacuna] and at least one sequence selected from SEQ ID NO 1-12, 15, 29 and 30 and represented as RNA, with the proviso that the transgenic organism is not a human.
